# EUROPÄISCHE PATENTSCHRIFT

(11) **EP 1 123 913 B1**
(45) Veröffentlichungstag und Bekanntmachung des Hinweises auf die Patenterteilung: **07.04.2004**
(21) Anmeldenummer: 01101658.1
(22) Anmeldetag: 29.01.2001
(51) Int. Cl.: C07C 41/16, C07C 253/30, C07C 255/37, C07C 45/64, C07C 47/575

(54) **Verfahren zur Herstellung von 2-Methoxyethoxy-benzolen und neue 2-Methoxyethoxy-benzylcyanide**
Process for preparing 2-methoxyethoxybenzenes and new 2-methoxyethoxybenzyl cyanides
Procédé pour la préparation de 2-méthoxyéthoxybenzènes; de nouveaux cyanures 2-méthoxyéthoxybenzyliques

(30) Priorität: 09.02.2000 DE 10005570
(43) Veröffentlichungstag der Anmeldung: 16.08.2001
(73) Patentinhaber: Bayer Chemicals AG, 51368 Leverkusen (DE)
(72) Erfinder: Giffels, Guido, Dr., 53177 Bonn (DE); Dreisbach, Claus, Dr., 51065 Köln (DE)

(56) Entgegenhaltungen:
- G. S. SHEPPARD: "3-(2-(3-Pyridinyl)thiazolidin-4-oyl)indol es, a novel series of platelet activating factor antagonists" JOURNAL OF MEDICINAL CHEMISTRY, Bd. 37, Nr. 13, 1994, Seiten 2011-2032, XP002170395 WASHINGTON US
- Z. BUDESINSKY: "6,7-Dialkoxychinoxalin-derivate" COLLECTION OF CZECHOSLOVAK CHEMICAL COMMUNICATIONS., Bd. 36, 1971, Seiten 2527-2539, XP002170396 PRAGUE CS
- REN-LI LI: "A comparison of the inhibitory action of 5-(substituted-benzyl)-2,4-diaminopyrimidi nes on dihydrofolate reductase from chicken liver with that from bovine liver" JOURNAL OF MEDICINAL CHEMISTRY, Bd. 25, Nr. 4, 1982, Seiten 435-440, XP002170397 WASHINGTON US

## Beschreibung

Die vorliegende Erfindung betrifft ein vorteilhaftes Verfahren zur Herstellung von 2-Methoxyethoxy-benzolen aus entsprechenden Phenolen und neue 2-Methoxyethoxy-benzylcyanide.

Alkoxybenzylcyanide wie 4-(2-Methoxyethoxy)-benzylcyanid sind Wirkstoffzwischenprodukte, die eine Reihe von Folgeprodukten, wie die entsprechenden Phenylessigsäuren, Amide und Amine zugänglich machen. Für das aus 4-(2-Methoxyethoxy)-benzylcyanid leicht erhältliche 2-(4-(2-Methoxyethoxy)-phenyl)-ethylamin sind Verwendungen als Baustein in der Synthese pharmakologisch wirksamer Substanzen bekannt (WO 98/37079). Die Synthese dieses Amins ist jedoch bisher nicht in der Literatur beschrieben. Das erfindungsgemäße 4-(2-Methoxyethoxy)-benzylcyanid ermöglicht einen einfachen Zugang zu diesem Synthesebaustein.

Es ist bekannt, dass man 3- und 4-(2-Methoxyethoxy)-benzaldehyd herstellen kann, indem man 3- bzw. 4-Hydroxybenzaldehyd mit 2-Chlorethyl-methyl-ether in Dimethylformamid in Gegenwart von Kaliumcarbonat am Rückfluss kocht. Man erzielt so Ausbeuten von 60,5 bzw. 75,5 % d.Th. (siehe J. Med. Chem. 25, 440 (1982) Methode B). Nachteilig ist zum einen die zum Teil unbefriedigende Ausbeute und zum anderen der Einsatz eines kostspieligen Lösungsmittels, das großen arbeitshygienischen Aufwand erfordert und nicht vollständig recyclisiert werden kann.

Bei einem Verfahren zur Herstellung von 2-Methoxyethoxy-benzol (dort als 1-Methyl-4-phenyl-ethylenglykol bezeichnet) wird Phenol mit 2-Bromethyl-methylether in Acetonitril in Gegenwart von Kaliumcarbonat durch Kochen am Rückfluss umgesetzt (siehe Can. J. Chem. 73, 572 (1995)). Neben dem Aufwand für die Bereitstellung der Bromverbindung und den Nachteilen durch den Einsatz von Acetonitril (die ähnlich sind wie beim Einsatz von Dimethylformamid) ist die erreichte Ausbeute von nur 26 % völlig unbefriedigend.

Weiterhin ist aus B. Budesinsky: '6,7-Dialkoxychinixalin-derivate', Collection of Czechoslovak Chemical Communications, Bd. 36, 1971, Seiten 2527-2539, bekannt, Phenole bzw. Phenolate mit Chlorethylmethylether in Methoxyethanol als Lösungsmittel zu 2-Methoxyethylphenolethern umzusetzen. Allerdings werden gemäß diesem Verfahren lediglich Ausbeuten von 72 bis 78 % realisiert, was für die technische Anwendbarkeit unbefriedigend ist.

Es besteht deshalb noch ein Bedürfnis nach einem Verfahren zur Herstellung von 2-Methoxyethoxy-benzolen, das einfach und kostengünstig die gewünschten Produkte in guten Ausbeuten liefert.

Es wurde nun ein Verfahren zur Herstellung von 2-Methoxyethoxy-benzolen aus den entsprechenden Phenolverbindungen durch Umsetzung mit 2-Chlorethyl-methyl-ether gefunden, das dadurch gekennzeichnet ist, dass man ohne Zusatz eines stark polaren Lösungsmittels bei Temperaturen über 95°C und unter Druck arbeitet.

Als Phenolverbindungen kann man beispielsweise solche der Formel (I) einsetzen 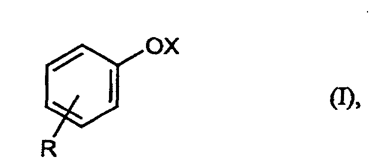 in der
- X: für H, ein Alkalimetall oder ein halbes Äquivalent eines Erdalkalimetalls und
- R: für H, CHO, CH₂CN, CN, NO₂, C₁-C₁₀-Alkyl, C₁-C₁₀-Alkoxy, (CH₂)ₓ-OH mit x = 1 bis 5, Halogen oder Halogen-C₁-C₁₀-alkyl stehen.

In Formel (I) befindet sich der Rest R vorzugsweise in ortho- oder para-Stellung, insbesondere in para-Stellung zur OX-Gruppe.

In Formel (I) steht X vorzugsweise für H, Na oder K.

Wenn in Formel (I) X für H steht wird das erfindungsgemäße Verfahren in Gegenwart einer Base durchgeführt, sonst ist die Gegenwart einer Base nicht zwingend, aber vorteilhaft. Als Basen kommen z.B. Hydride, Hydroxide, Oxide, Alkoholate, Hydrogencarbonate und Carbonate von Alkali- und Erdalkalimetallen in Frage. Bevorzugt sind Kalium- und Natriumcarbonat.

Wenn man in Gegenwart von Basen arbeitet, können pro Mol Phenolverbindung der Formel (I) beispielsweise 0,5 bis 10 Äquivalente, vorzugsweise 0,9 bis 2 Äquivalente einer oder mehrerer Basen eingesetzt werden.

In Formel (I) steht R vorzugsweise für H, CHO, CH₂CN oder CF₃.

2-Chlorethyl-methyl-ether kann man beispielsweise in Mengen von 1 bis 50 Mol pro Mol Phenolverbindung einsetzen. Vorzugsweise liegt diese Menge bei 2 bis 20 Mol.

Es ist ein wesentliches Merkmal des erfindungsgemäßen Verfahrens, dass kein stark polares Lösungsmittel wie Dimethylformamid oder Acetonitril eingesetzt wird. Beispiele für Lösungsmittel, die beim erfindungsgemäßen Verfahren zugegen sein können sind überschüssiger 2-Chlorethyl-methyl-ether oder schwach polare oder unpolare Lösungsmittel wie gegebenenfalls halogenierte aromatische Kohlenwasserstoffe, beispielsweise Toluol, Xylole, Chlorbenzol und Dichlorbenzole. Überschüssiger 2-Chlorethyl-methyl-ether ist bevorzugt.

Das erfindungsgemäße Verfahren kann man z.B. bei Temperaturen im Bereich 95 bis 300°C durchführen. Vorzugsweise arbeitet man bei 100 bis 200°C, insbesondere bei 130 bis 170°C.

Es ist ein weiteres wesentliches Merkmal des erfindungsgemäßen Verfahrens, dass man es unter Druck durchführt. Der Druck kann z.B. im Bereich 1 bis 60 bar liegen.

Vorzugsweise arbeitet man in einem geschlossenen Gefäß, bei dem sich bei der jeweiligen Reaktionstemperatur von selbst einstellenden Druck. Bei den besonders bevorzugten Reaktionstemperaturen ergibt das Drücke unter 10 bar, häufig unter 6 bar.

Die Umsetzung der Phenolverbindungen mit dem 2-Chlorethyl-methyl-ether ist im allgemeinen nach 3 bis 20 Stunden beendet. Selbstverständlich ist es günstig, während der Umsetzung zu rühren.

Das nach Durchführung des erfindungsgemäßen Verfahrens vorliegende Reaktionsgemisch kann man beispielsweise aufarbeiten, indem man es abkühlt, die festen Bestandteile des Reaktionsgemisches (im wesentlichen Chloride und gegebenenfalls Basen) z.B. durch Filtration abtrennt und aus dem Filtrat zunächst gegebenenfalls vorhandenen überschüssigen 2-Chlorethyl-methyl-ether und/oder Lösungsmittel und anschließend die hergestellten 2-Methoxyethoxy-benzole, z.B. durch fraktionierte Destillation gewinnt. Der abgetrennte 2-Chlorethyl-methyl-ether ist von hoher Reinheit, so dass er vollständig und vielfach recyclisiert werden kann.

Es ist möglich, dass das hergestellte 2-Methoxyethyl-benzol in manchen Fällen noch geringe Mengen unumgesetzte Phenolverbindung enthält. Gewünschtenfalls kann man diese entfernen, z.B. durch Verdünnen mit einem inerten Lösungsmittel, z.B. Toluol, und Extraktion mit einer starken Base, z.B. wässriger Natronlauge.

Die erfindungsgemäß herzustellenden 2-Methoxyethoxy-benzole entsprechen z.B. der Formel (II) 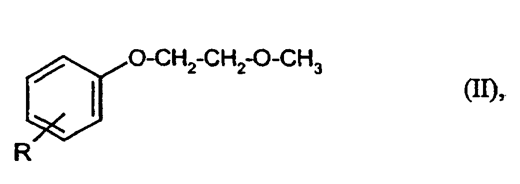 in der
- R: die bei Formel (I) angegebene Bedeutung hat.

Das erfindungsgemäße Verfahren hat eine Reihe von Vorteilen. So sind die Ausbeuten und Reinheiten der erhältlichen 2-Methoxyethoxy-benzole hoch. Häufig liegt die Ausbeute um 90 % d.Th. und häufig deutlich höher als gemäß dem Stand der Technik. Die Reinheit der erhaltenen 2-Methoxyethoxy-benzole liegt häufig bei 98 % und kann bis nahezu 100 % erreichen. Kostspielige, hohen arbeitshygienischen Aufwand erfordernde und nicht vollständig recyclisierbare Lösungsmittel wie Dimethylformamid werden nicht benötigt. Zur Durchführung des erfindungsgemäßen Verfahrens reichen Rührwerkskessel aus, die für Drücke von 1 bis 60 bar ausgelegt sind. Die Reaktionszeiten sind in der Regel kürzer als bei bekannten Verfahren.

Diese Vorteile sind überraschend, denn erfindungsgemäß wird bei ähnlich hohen Temperaturen gearbeitet wie gemäß dem Stand der Technik. Bei ähnlichen Temperaturen muss jedoch hier wie dort mit Nebenreaktionen gerechnet werden, die die Ausbeute und Reinheit der hergestellten Produkte negativ beeinflussen, insbesondere beim Einsatz von Phenolverbindungen der Formel (I) mit R ungleich H. Überraschenderweise sind beim erfindungsgemäßen Verfahren die Ausbeuten und Reinheiten besser. Ferner war nicht vorauszusehen, dass das Weglassen der bisher für notwendig erachteten stark polaren Lösungsmittel sich nicht negativ auswirkt.

Die vorliegende Erfindung betrifft weiterhin neue 2-Methoxyethoxy-benzylcyanide der Formel (III) 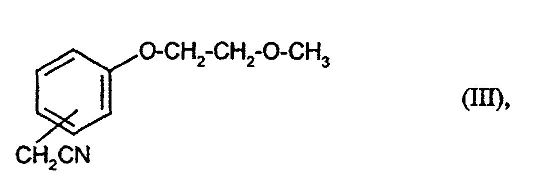 in der
die CH₂CN-Gruppe in ortho- oder para-Stellung zur 2-Methoxyethoxy-Gruppe angeordnet ist.

Die Herstellung und Verwendung dieser neuen Verbindungen ist oben beschrieben worden. Die neuen Verbindungen bereichern die Technik insbesondere dadurch, dass neue Spezies aus der Gruppe der 2-Methoxyethoxy-benzole zur Verfügung gestellt werden. Damit wird die Palette von Zwischenprodukten erweitert und Herstellmöglichkeiten für potentielle neue Wirkstoffe eröffnet.

Ferner kann man durch Hydrierung von 4-(2-Methoxyethoxy)-benzylcyanid z.B. in Gegenwart von Raney-Nickel und Ammoniak in Isopropanol bei 80°C und 50 bar Wasserstoffdruck 2-(4-(2-Methoxyethoxy)-phenyl)-ethylamin in Ausbeuten von über 90 % erhalten. Dieses Amin war bisher, trotz Nennung in WO 98/37079, nicht verfügbar, weil kein Herstellungsverfahren bekannt war.

4-(2-Methoxyethoxy)-benzylcyanid ist auch durch Chlormethylierung von 1-(2-Methoxyethoxy)-benzol zu 4-(2-Methoxyethoxy)-benzylchlorid und dessen Umsetzung mit Cyanid zugänglich.

### Beispiele

### Beispiel 1

### 1-(2-Methoxyethoxy)-benzol

In einem 31-Autoklaven mit Blattrührer wurden 209 g Natriumphenolat und 10 g Kaliumcarbonat in 1100 g 2-Chlorethyl-methyl-ether vorgelegt. Der Autoklav wurde verschlossen, mit Stickstoff gespült und unter Rühren innerhalb von 1 Stunde auf 150°C aufgeheizt. Man rührte 10 Stunden bei dieser Temperatur, wobei der Innendruck auf maximal 5,4 bar anstieg. Dann wurde abgekühlt, die vorhandenen Salze abfiltriert und mit 48,5 g 2-Chlorethyl-methyl-ether nachgewaschen. Die vereinigten Filtrate wurden unter verringertem Druck eingedampft, wobei 808,8 g 2-Chlorethyl-methyl-ether mit einer Reinheit von 98,6 % (GC-Flächen-%) als Destillat erhalten wurden. Der flüssige Rückstand, der laut GC noch etwa 2 % Phenol enthielt, wurde mit 387 g Toluol verdünnt und zweimal mit je 500 g 10 %iger wässriger Natronlauge gewaschen. Die organische Phase wurde dann am Rotationsverdampfer eingedampft und der Rückstand von 259,6 g im Vakuum fraktioniert destilliert. Bei 9 mbar gingen bei einer Kopftemperatur von 90 bis 93°C 213 g reines Produkt in Form einer klaren, farblosen Flüssigkeit über, entsprechend einer Ausbeute von 78 %. Mittels GC konnten keinerlei Nebenprodukte gefunden werden.

### Beispiel 2

### 4-(2-Methoxyethoxy)-benzaldehyd

36,63 g 4-Hydroxybenzaldehyd wurden mit 60 g Kaliumcarbonat und 189,08 g 2-Chlorethyl-methyl-ether in einem 0,71-Autoklaven 10 Stunden bei 150°C gerührt. Nach Filtration, Wäsche mit 50 g 2-Chlorethyl-methyl-ether und Abdestillieren des überschüssigen 2-Chlorethyl-methyl-ethers analog Beispiel 1 wurden 50,8 g Produkt in Form einer klaren, rötlichen Flüssigkeit und 106,23 g 2-Chlorethyl-methyl-ether mit einer Reinheit von 99,8 % laut GC erhalten. Das Produkt wies einen Gehalt von 98,3 Gew.-% laut HPLC auf, entsprechend einer Ausbeute von 92,4 %.

### Beispiel 3

### 4-(2-Methoxyethoxy)-benzaldehyd

219,8 g 4-Hydroxybenzaldehyd wurden mit 270 g Kaliumcarbonat und 1134 g 2-Chlorethyl-methyl-ether in einem 3 1-Autoklaven 12 Stunden bei 150°C gerührt. Nach Filtration, Wäsche mit 46,1 g 2-Chlorethyl-methyl-ether und Abdestillieren des überschüssigen 2-Chlorethyl-methyl-ethers analog Beispiel 1 wurden 319,9 g Rohprodukt als rotbraune Flüssigkeit und 819,2 g 2-Chlorethyl-methyl-ether mit einer Reinheit von 99,4 % (GC) erhalten. Die Fraktionierung des Rohproduktes bei 1 mbar lieferte bei 117 bis 120°C 281,2 g hochreines Produkt in Form einer klaren, farblosen Flüssigkeit, entsprechend einer Ausbeute von 87 %.

### Beispiel 4

### 4-(2-Methoxyethoxy)-benzaldehyd

65 g 6-Hydroxybenzaldehyd wurden in 350 g 2-Chlorethylmethylether suspendiert und mit 96 g 30 %iger Natriummethanolatlösung in Methanol versetzt. Das Methanol wurde abdestilliert und die erhaltene Suspension 10 Stunden bei 150°C im Autoklaven gerührt. Nach Filtration, Wäsche mit 10,8 g 2-Chlorethylmethylether und Abdestillieren des überschüssigen Reagenzes wurden 237,5 g 2-Chlorethylmethylether als Destillat in einer Reinheit von 99 % laut GC und 82,7 g Rohprodukt als rötlichbraune Flüssigkeit erhalten. Das Rohprodukt enthielt neben restlichem 4-Hydroxybenzaldehyd laut HPLC-Analyse 87,9 Gew.-% des Produktes, entsprechend einer Ausbeute von 76 % d. Th.

### Beispiel 5

### 4-(2-Methoxyethoxy)-benzylcyanid

30 g 4-Hydroxybenzylcyanid wurden mit 34,25 g Kaliumcarbonat und 189,1 g 2-Chlorethyl-methyl-ether 10 Stunden bei 150°C in einem 0,71-Autoklaven gerührt. Nach Filtration, Wäsche mit 20,5 g 2-Chlorethyl-methyl-ether und Abdestillieren des überschüssigen 2-Chlorethyl-methyl-ethers analog Beispiel 1 wurden 139,3 g 2-Chlorethyl-methyl-ether als Destillat mit einem Gehalt von 98,4 % (GC) und 41,7 g Produkt in Form eines Öles, das beim Stehen spontan kristallisierte, erhalten. Das Produkt zeigte im GC-MS und ¹H-NMR keine Verunreinigungen. Die ¹H-NMR-Untersuchung (CDCl₃, 400 MHz) ergab folgende charakteristische Signale: δ = 3,45 (s, 3H); 3,68 (s, 2H); 3,77 (t, 2H); 4,12 (t, 2H); 6,91 (2H) und 7,23 (2H). Die Ausbeute betrug 96,8 %.

### Beispiel 6

### 4-(2-Methoxyethoxy)-benzaldehyd

157 g 4-Hydroxybenzaldehyd wurden unter Stickstoff in 540ml Methanol gelöst vorgelegt. Zu der Lösung wurden portionsweise über 1,5 Stunden 51,4 g Natriumhydroxid gegeben (exotherm). Es wurde noch 1,5 Stunden nachgerührt, dann wurde das Lösungsmittel abdestilliert. Der Rückstand (4-Hydroxybenzaldehyd-Na-Salz, 212 g) wurde mit 810 g 2-Chlorethylmethylether und 1 g Kaliumiodid in einem 3 Liter-Autoklaven 10 Stunden bei 150°C gerührt. Danach wurde der Autoklaveninhalt entnommen, der Autoklav mit 47,2 g 2-Chlorethylmethylether nachgewaschen und der 2-Chlorethylmethylether abdestilliert. Es wurden 689,3 g 2-Chlorethylmethylether mit einem GC-Gehalt von 99,24 Flächen-% wiedergewonnen. Der Rückstand wurde in 340 ml Toluol aufgenommen und mit 500 g 10%iger wässriger Natronlauge extrahiert. Es wurden 499 g organische Phase erhalten, die Wasserphase wurde verworfen.

Die Analyse der organischen Phase ergab einen Gehalt von 42,5 Gew.-% Produkt entsprechend einer Ausbeute von 91,5 %.

## Patentansprüche

1. Verfahren zur Herstellung von 2-Methoxyethoxy-benzolen aus den entsprechenden Phenolverbindungen durch Umsetzung mit 2-Chlorethyl-methyl-ether, **dadurch gekennzeichnet, dass** man überschüssigen 2-Chlorethyl-methyl-ether als Lösungsmittel einsetzt und bei Temperaturen zwischen 95 und 300°C und unter einem Druck von 1 bis 60 bar arbeitet.

2. Verfahren nach Anspruch 1, **dadurch gekennzeichnet, dass** man als Phenolverbindungen solche der Formel (I) einsetzt 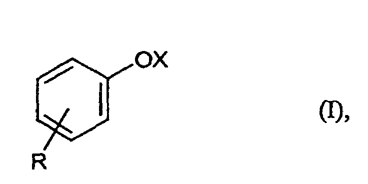 in der
X für H, ein Alkalimetall oder ein halbes Äquivalent eines Erdalkalimetalls und
R für H, CHO, CH₂CN, CN, NO₂, C₁-C₁₀-Alkyl, C₁-C₁₀-Alkoxy, (CH₂)ₓ-OH mit x = 1 bis 5, Halogen oder Halogen-C₁-C₁₀-alkyl stehen.

3. Verfahren nach Anspruch 2, **dadurch gekennzeichnet, dass** sich in Formel (I) der Rest R in ortho- oder para-Stellung zur OX-Gruppe befindet.

4. Verfahren nach Ansprüchen 1 bis 3, **dadurch gekennzeichnet, dass** man es in Gegenwart einer Base durchführt.

5. Verfahren nach Ansprüchen 1 bis 4, **dadurch gekennzeichnet, dass** man das nach der Umsetzung vorliegende Reaktionsgemisch aufarbeitet, indem man es abkühlt, die festen Bestandteile durch Filtration abtrennt und aus dem Filtrat zunächst gegebenenfalls vorhandenen überschüssigen 2-Chlorethyl-methyl-ether und/oder Lösungsmittel und anschließend das hergestellte 2-Methoxyethoxy-benzol durch fraktionierte Destillation gewinnt.

6. Verbindungen der Formel (III) 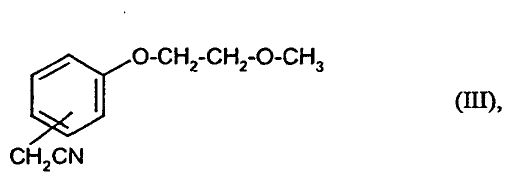 in der
die CH₂CN-Gruppe in ortho- oder para-Stellung zur 2-Methoxyethoxy-Gruppe angeordnet ist.

## Claims

1. Process for the preparation of 2-methoxyethoxy-benzenes from the corresponding phenol compounds by reaction with 2-chloroethyl methyl ether, **characterized in that** excess 2-chloroethyl methyl ether is used as solvent and the process is carried out at temperatures between 95 and 300°C and under a pressure of from 1 to 60 bar.

2. Process according to Claim 1, **characterized in that** the phenol compounds used are those of the formula (I) 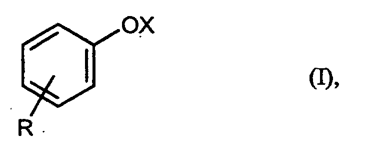 in which
X is H, an alkali metal or a half-equivalent of an alkaline earth metal and
R is H, CHO, CH₂CN, CN, NO₂, C₁-C₁₀-alkyl, C₁-C₁₀-alkoxy, (CH₂)ₓ-OH where x = 1 to 5, halogen or halogeno-C₁-C₁₀-alkyl.

3. Process according to Claim 2, **characterized in that**, in formula (I), the radical R is in the ortho- or para-position relative to the OX group.

4. Process according to Claims 1 to 3, **characterized in that** it is carried out in the presence of a base.

5. Process according to Claims 1 to 4, **characterized in that** the reaction mixture present after the reaction is worked up by cooling it, removing the solid constituents by filtration and, from the filtrate, obtaining firstly any excess 2-chloroethyl methyl ether and/or solvent which may be present and then the prepared 2-methoxyethoxy-benzene by fractional distillation.

6. Compounds of the formula (III) 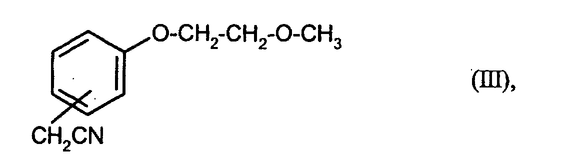 in which
the CH₂CN group is arranged in the ortho- or para-position relative to the 2-methoxyethoxy group.

## Revendications

1. Procédé de préparation de 2-méthoxyéthoxybenzènes à partir des composés phénoliques correspondants par mise en réaction de 2-chloréthylméthyléther, **caractérisé en ce que** l'on utilise du 2-chloréthylméthyléther excédentaire comme solvant à des températures entre 95°C et 300°C et à une pression de 1 à 60 bars.

2. Procédé selon la revendication 1, **caractérisé en ce que** les composés phénoliques utilisés sont de formule (I) 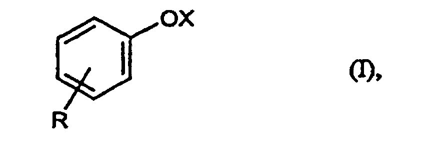 dans laquelle
X désigne un métal alcalin ou un demi-équivalent d'un métal alcalino-terreux et
R désigne H, CHO, CH₂CN, CN, NO₂, un radical alkyle C₁-C₁₀, alcoxy C₁-C₁₀, (CH₂)ₓ-OH avec x = 1 à 5, un halogène ou un radical halogéno-C₁-C₁₀-alkyle.

3. Procédé selon la revendication 2, **caractérisé en ce que** le radical R se trouve en position ortho ou para par rapport au radical OX dans la formule (I).

4. Procédé selon l'une des revendications 1 à 3, **caractérisé en ce que** l'on procède en présence d'une base.

5. Procédé selon l'une des revendications 1 à 4, **caractérisé en ce que** le mélange réactif obtenu après la mise en réaction est traité en le refroidissant, en séparant les composants solides par filtration et, en obtenant d'abord à partir du filtrat le 2-chloréthylméthyléther et/ou le solvant excédentaire éventuellement présent et ensuite le 2-méthyléthoxybenzène, préparé par distillation fractionnée.

6. Composés de formule (III) 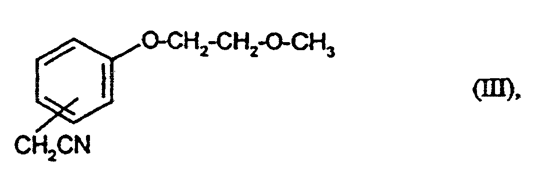 dans laquelle
le radical CH₂CN est disposé en position ortho ou para par rapport au radical 2-méthoxyéthoxy.
